# DEMANDE DE BREVET EUROPEEN

(11) **EP 1 845 082 A1**
(43) Date de publication de la demande: **17.10.2007**
(21) Numéro de dépôt: 07290439.4
(22) Date de dépôt: 11.04.2007
(51) Int. Cl.: C07C 309/05, C07C 309/14, C07C 309/17, C07C 309/29, C07C 309/46, A61K 31/185, A61P 19/10

(54) **Nouveaux sels de strontium d'acides sulfoniques, leur procede de preparation et les compositions pharmaceutiques qui les contiennent**

(30) Priorité: 12.04.2006 FR 0603224
(71) Demandeur: Les Laboratoires Servier, 92415 Courbevoie Cedex (FR)
(72) Inventeur: Lefeulon, François, 45000 Orleans (FR); Rolland, Yves, 92170 Vanves (FR)

(57) **Abrégé**

Sels de strontium des acides sulfoniques de fomule (I)
A-B-SO₃H (I)
dans laquelle:
- A représente un groupement choisi parmi OH, NH₂, SO₃H et CO₂H,
- B représente un groupement arylène ou une chaîne alkylène linéaire ou ramifiée C₁-C₁₂ éventuellement substituée et dont un ou plusieurs des atomes de carbone est éventuellement remplacé par un atome d'oxygène, un atome d'azote ou un groupement SO₂.
Médicaments en particulier utiles dans la prévention et le traitement de l'arthrose et l'ostéoporose.

## Description

La présente invention concerne de nouveaux sels de strontium d'acides sulfoniques, leur procédé de préparation et les compositions pharmaceutiques qui les contiennent.

De nombreux sels minéraux et organiques de strontium sont déjà connus dans la littérature. Parmi les dérivés d'acide sulfonique, le bis(aminométhanesulfonate) de strontium, ainsi que son utilisation dans les papiers photographiques, ont été décrits dans le brevet US 4,419,433. Le méthanedisulfonate de strontium a été décrit dans la publication Recueil : Journal of the Royal Netherlands Chemical Society 1981, 100(12), 449-452. Le *m-*benzènedisulfonate de strontium a été décrit dans la publication J.Phys. Chem. 1963, 67, 337-339. Le 6-sulfonatohexanoate de strontium a été décrit dans la publication Berichte 1897, 63, 1642-48. Le 1,5-naphtalènedisulfonate de strontium a été décrit dans la publication Acta Cryst. 2001, 57(4), 520-530. Le 1,6-naphtalènedisulfonate de strontium a été décrit dans la publication Helv. Chim. Acta 1923, 6(1), 1133-1146. Le bis (6-hydroxy-2-naphtalènesulfonate) de strontium a été décrit dans la publication Helv. Chim. Acta 1925, 8(1), 229-241. Le 2-oxo-1,3-propanedisulfonate de strontium a été décrit dans Therm. Anal. 1980, 6th, Vol 2, 425-430. L'hydroxyméthanesulfonate de strontium a été décrit dans le brevet JP 37006516. Le bis(4-aminobenzènesulfonate) de strontium a été décrit dans les publications J. Applied Polymer Sc. 2000, 77(11), 2363-69 et J. Prakt. Chem. 1934, 245-253. Le bis (2-amino-benzènesulfonate) de strontium et le bis (3-aminobenzènesulfonate) de strontium ont été décrits dans la publication J. Prakt. Chem. 1935, 6-10. Le 1,2-éthanedisulfonate de strontium a été décrit dans la publication Kristallogr. Kristallgeom. Kristallchem. 1913, 51, 502. Le 3-amino-3-carboxy-1-propanesulfonate chlorure de strontium a été décrit dans la publication Inorg. Chem. 2005, 44(11), 3890-95.

L'utilisation de sels de strontium à usage thérapeutique a également fait l'objet de publications et de brevets. A titre d'exemple, le brevet US 4,152,431 décrit des sels de métaux alcalins utilisables dans le traitement de l'inflammation. La demande de brevet WO 94/09798 présente des complexes de sulfates de différents métaux, actifs dans le traitement des maladies de la peau. Les travaux de Olle Svensson et coll. (Acta Path. Microbiol. Immunol. Scand., Sect. A 1985, 93, 115-120) montrent que le strontium joue un rôle dans certains cas de rachitisme.

Le ranélate de strontium, ainsi que son utilisation dans le traitement de l'ostéoporose, ont été décrits dans le brevet EP 0 415 850.

La présente invention concerne de nouveaux sels de strontium dérivés d'acide sulfonique, ainsi que leur utilisation dans le traitement de l'arthrose et de l'ostéoporose.

L'ostéoporose est une diminution de la minéralisation calcique au niveau du squelette. Comme tous les tissus vivants, l'os est soumis à un processus continuel de destruction-reconstruction : c'est le remodelage osseux. Il se caractérise d'abord par une phase de résorption de la matrice osseuse ancienne par les ostéoclastes, suivie par une phase de réparation par la formation de la matrice protéique par les ostéoblastes, qui sera ensuite minéralisée. Chez le sujet jeune, le bilan osseux est équilibré, mais avec l'âge apparaît un déséquilibre entre résorption et formation osseuse au détriment de cette dernière.

L'arthrose est une pathologie dégénérative qui touche les cartilages articulaires dans plus de 50 % de la population après 65 ans. Elle se caractérise toujours par une dégradation de la matrice cartilagineuse qui entoure les chondrocytes. Dans des conditions non pathologiques, ces cellules assurent l'homéostasie du cartilage. En revanche, sous l'effet d'un certain nombre de facteurs (biomécaniques et/ou biochimiques) les chondrocytes sont à l'origine de la dégradation de ce tissu environnant car elles sécrètent alors des métalloprotéases qui dégradent le collagène de type II et les protéoglycanes qui sont les composants matriciels caractéristiques du cartilage.

Plus spécifiquement, la présente invention concerne les sels de strontium des acides sulfoniques de formule (I) :

A-B-SO₃H (I)

dans laquelle :
- A représente un groupement choisi parmi OH, NH₂, SO₃H et CO₂H,
- B représente un groupement arylène ou une chaîne alkylène linéaire ou ramifiée C₁-C₁₂ éventuellement substituée par un ou plusieurs groupements choisis parmi hydroxy, oxo, amino, SO₃H, CO₂H et dont un ou plusieurs des atomes de carbone est éventuellement remplacé par un atome d'oxygène, par un atome d'azote ou un groupement SO₂,
à l'exclusion du bis(aminométhanesulfonate) de strontium, du méthanedisulfonate de strontium, du 1,3-benzènedisulfonate de strontium, du 6-sulfonatohexanoate de strontium, du 1,5-naphtalènedisulfonate de strontium, du 1,6-naphtalènedisulfonate de strontium, du bis (6-hydroxy-2-naphtalènesulfonate) de strontium, du 2-oxo-1,3-propanedisulfonate de strontium, de l'hydroxyméthanesulfonate de strontium, du bis(4-aminobenzènesulfonate) de strontium, du bis (2-amino-benzènesulfonate) de strontium, du bis (3-aminobenzènesulfonate) de strontium, du 1,2-éthanedisulfonate de strontium et du 3-amino-3-carboxy-1-propanesulfonate chlorure de strontium.

Par arylène, on entend phénylène, biphénylylène ou naphtylène, chacun de ces groupements étant éventuellement substitué par un ou plusieurs groupements, identiques ou différents, choisis parmi alkyle C₁-C₆ linéaire ou ramifié, OH, NH₂, SO₃H et CO₂H.

Un aspect de la présente invention concerne les sels de strontium des composés de formule (I) pour lesquels A représente NH₂ ou SO₃H.

Un autre aspect de la présente invention concerne les sels de strontium des composés de formule (I) pour lesquels B représente une chaîne alkylène linéaire C₁-C₆, substituée ou non substituée.

Un autre aspect de la présente invention concerne les invention concerne les sels de strontium des composés de formule (I) pour lesquels B représente une chaîne alkylène linéaire C₃-C₆, substituée ou non substituée.

Un autre aspect de la présente invention concerne les sels de strontium des acides sulfoniques de formule (Ia), cas particulier des acide sulfoniques de formule (I) :

A-B-SO₃H (Ia)

dans laquelle :
- A représente un groupement choisi parmi NH₂ et SO₃H,
- B représente une chaîne alkylène linéaire ou ramifiée C₃-C₆ non substituée.

Un autre aspect de la présente invention concerne les sels de strontium suivants :
- le 1,3-propanedisulfonate de strontium;
- le bis(3-amino-1-propanesulfonate) de strontium;
- le sulfonatoacétate de strontium;
- le bis(2-amino-1-éthanesulfonate) de strontium;
- le 1,4-butanedisulfonate de strontium; et
- le 1,2-benzènedisulfonate de strontium.

L'invention s'étend également à un premier procédé de préparation des sels de strontium selon l'invention par réaction d'un acide sulfonique de formule (I) :

A-B-SO₃H (I)

dans laquelle A et B sont tels que définis précédemment,
avec de l'hydroxyde de strontium, suivie de l'isolement du sel de strontium ainsi obtenu.

L'invention s'étend également à un deuxième procédé de préparation des sels de strontium selon l'invention par réaction du sel de sodium ou de potassium d'un acide sulfonique de formule (I) :

A-B-SO₃H (I)

dans laquelle A et B sont tels que définis précédemment,
avec du chlorure de strontium, suivie de l'isolement du sel de strontium ainsi obtenu.

L'invention s'étend aussi aux compositions pharmaceutiques renfermant comme principe actif un sel de strontium selon l'invention avec un ou plusieurs excipients inertes, non toxiques et appropriés tels que des diluants, des lubrifiants, des liants, des agents de désintégration, des absorbants, des colorants, des édulcorants.

A titre d'exemple et de manière non limitative, on peut citer :
- *pour les diluants :* le lactose, le dextrose, le sucrose, le mannitol, le sorbitol, la cellulose, la glycérine,
- *pour les lubrifiants* : la silice, le talc, l'acide stéarique et ses sels de magnésium et de calcium, le polyéthylène glycol,
- *pour les liants :* le silicate d'aluminium et de magnésium, l'amidon, la gélatine, la tragacanthe, la méthylcellulose, la carboxyméthylcellulose de sodium, le carboxyméthylamidon et la polyvinylpyrrolidone,
- *pour les désintégrants* : l'agar, l'acide alginique et son sel de sodium, les mélanges effervescents.

Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables.

La posologie utile varie selon le sexe, l'âge et le poids du patient, la voie d'administration, la nature de l'affection et des traitements éventuellement associés et s'échelonne entre 5 mg et 3 g par 24 heures, par exemple entre 100 mg et 2 g par 24 heures.

Les exemples suivants illustrent l'invention.

### EXEMPLE 1 : 1,3-Propanedisulfonate de strontium

Un mélange de 222g d'acide 1,3-propanedisulfonique à 70% dans l'eau (0,76 mol) et 200,4g d'hydroxyde de strontium octahydrate (0,75 mol) dans 200 ml d'eau est chauffé à reflux pendant 3h. Le mélange réactionnel est filtré à chaud puis refroidi à 4°C. Le précipité qui se forme est éliminé par filtration et 500 mL d'éthanol à 96% sont ajoutés au filtrat. Le précipité est filtré, rincé avec 250 mL d'un mélange éthanol/eau 70/30 et séché pour obtenir 139g d'un produit cristallin blanc de sel de strontium d'acide 1,3-propanedisulfonique (rendement 63%).
*Température de fusion :* >250°C

**Microanalyse élémentaire:**

| | %C | %H | %S | %Sr |
|---|---|---|---|---|
| Calculé | 12,43 | 2,09 | 22,13 | 30,23 |
| Trouvé | 12,56 | 3,44 | 22,76 | 26,88 |

### EXEMPLE 2 : Bis(3-amino-1-propanesulfonate) de strontium

Le produit attendu est obtenu par réaction de l'acide 3-amino-1-propanesulfonique avec l'hydroxyde de strontium octahydrate selon le procédé de l'Exemple 1.

### EXEMPLE 3 : Sulfonatoacétate de strontium

Le produit attendu est obtenu par réaction de l'acide sulfoacétique avec l'hydroxyde de strontium octahydrate selon le procédé de l'Exemple 1.
*Température de fusion :* >270°C

**Microanalyse élémentaire:**

| | %C | %H | %S |
|---|---|---|---|
| Calculé | 10,64 | 0,89 | 14,21 |
| Trouvé | 9,77 | 1,20 | 14,18 |

### EXEMPLE 4 : Bis(2-amino-1-éthanesulfonate) de strontium

Le produit attendu est obtenu par réaction de l'acide 2-amino-1-éthanesulfonique avec l'hydroxyde de strontium octahydrate selon le procédé de l'Exemple 1.

### EXEMPLE 5 : 1,4-Butanedisulfonate de strontium

0,75 mol de chlorure de strontium hexahydrate et 0,75 mol de 1,4-butanedisulfonate de disodium sont dissous dans 600 ml d'eau. Après 1h d'agitation, 1 1 d'éthanol à 96% est ajouté. Le précipité qui se forme est filtré, rincé avec 500 ml d'un mélange éthanol/eau 70/30 et séché pour conduire produit attendu.

### EXEMPLE 6 : 1,2-Benzènedisulfonate de strontium

Le produit attendu est obtenu par réaction du 1,2-benzènedisulfonate de dipotassium avec le chlorure de strontium hexahydrate selon le procédé de l'Exemple 6.

### EXEMPLE 7 : Bis(2-hydroxyéthanesulfonate) de strontium

Le produit attendu est obtenu par réaction du 2-hydroxyéthanesulfonate de sodium avec le chlorure de strontium hexahydrate selon le procédé de l'Exemple 6.

### EXEMPLE 8 : 2,2-Bis(sulfonatométhyl)-1,3-propanedisulfonate de distrontium

Le produit attendu est obtenu par réaction de l'acide 2,2-bis(sulfométhyl)-1,3-propanedisulfonique avec l'hydroxyde de strontium octahydrate selon le procédé de l'Exemple 1.

### EXEMPLE 9 : 1,5-Pentanedisulfonate de strontium

Le produit attendu est obtenu par réaction du 1,5-pentanedisulfonate de disodium avec le chlorure de strontium hexahydrate selon le procédé de l'Exemple 6.

### EXEMPLE 10 : 1,6-Hexanedisulfonate de strontium

Le produit attendu est obtenu par réaction du 1,6-hexanedisulfonate de disodium avec le chlorure de strontium hexahydrate selon le procédé de l'Exemple 6.

### EXEMPLE 11 : Bis(4-amino-1-butanesulfonate) de strontium

Le produit attendu est obtenu par réaction de l'acide 4-amino-1-butanesulfonique avec l'hydroxyde de strontium octahydrate selon le procédé de l'Exemple 1.

### EXEMPLE 12 : Bis(1-amino-2-benzènesulfonate) de strontium

Le produit attendu est obtenu par réaction de l'acide 1-amino-2-benzènesulfonique avec l'hydroxyde de strontium octahydrate selon le procédé de l'Exemple 1.

### ETUDE PHARMACOLOGIQUE

### EXEMPLE 13 : Etude in vitro de l'inhibition du catabolisme de cartilage bovin par le composé de l'Exemple 1.

### Protocole

L'étude a été réalisée sur des fragments de cartilage bovin en milieu de culture (plaque de 96 puits), dont la dégradation est stimulée par l'ajout de TNFα et d'oncostatine M ( Schaller, S., Henriksen, K., Hoegh-Andersen, P., Sondergaard, B.C., Sumer, E.U., Tanko, L.B., Qvist, P. Karsdal, M.A. In vitro, ex vivo, and in vivo methodological approaches for studying therapeutic targets of osteoporosis and degenerative joint diseases: how biomarkers can assist ? Assay. Drug Dev. Technol. 3, 553-580 (2005).

La durée de la période de culture est de 21 jours (changement de milieu de culture tous les 2 jours).
Un groupe non traité par TNFα et oncostatine M constitue le groupe contrôle.
Cinq lots de fragments de cartilage stimulés ont été traités par le composé de l'Exemple 1, chacun à une dose différente : 0,01; 0,1; 1; 3 et 10 mM. Pour chaque dose, 5 réplications ont été effectuées.
A 119, on mesure la concentration de CTX II (fragment du collagène de type II issu de la dégradation de ce collagène par les métalloprotéases) dans le milieu de culture, par une technique d'ELISA. Ce paramètre est exprimé en ng/mL/mg de cartilage.
A J21, on mesure dans le cartilage restant en fin de culture:
- la quantité de protéines avec le kit commercialisé par Biorad. Pour les groupes traités par le composé de l'Exemple 1, les résultats de ce paramètre sont exprimés en pourcentage d'inhibition de la dégradation des protéines dans le cartilage du groupe témoin non traité.
- et la quantité de glycosaminoglycanes (GAGs), par la technique colorimétrique au bleu de méthylène (DMB). Ce paramètre est lui aussi exprimé en pourcentage d'inhibition de la dégradation des GAGs évaluée dans le groupe contrôle après 21 jours de culture.

### Résultats

Le traitement par le composé de l'Exemple 1 a pour effets :
- une inhibition significative (p< 0.001) et dose-dépendante du relargage de CTX II dans le milieu de culture (IC₅₀=3,5 mM);
- une protection dès la dose de 1mM de la dégradation des composants du cartilage :

| Composants | Dose (mM) | | | | |
|---|---|---|---|---|---|
| | 0,01 | 0,1 | 1 | 3 | 10 |
| protéines | / | / | 22 % | 41 % | 55 % * |
| GAGs | / | / | 4 % | 8 % | 26 % ** |

| | | | | | |
|---|---|---|---|---|---|
| * : p< 0,05 ; ** : p<0.01 | | | | | |

Le traitement a par ailleurs été bien toléré par les cellules durant les 21 jours de culture (test de toxicité utilisé : Alamar blue - négatif quelle que soit la dose).

Ces résultats démontrent que le composé de l'Exemple 1 protège significativement de la dégradation des composants caractéristiques du cartilage, via une inhibition de l'activité collagénolytique.

### EXEMPLE 14 : Composition pharmaceutique

**Formule de préparation pour un comprimé d'1 g dosé à 500 mg :**

| | |
|---|---|
| Composé de l'exemple 1 | 500 mg |
| Povidone K30 | 24 mg |
| Cellulose Avicel PM102 | 417 mg |
| Carboxyméthylamidon Primojel | 21 mg |
| Stéarate de magnésium | 6 mg |
| Talc | 32 mg |

## Revendications

1. Sels de strontium des acides sulfoniques de formule (I) :
A-B-SO₃H (I)
dans laquelle :
- A représente un groupement choisi parmi OH, NH₂, SO₃H et CO₂H,
- B représente un groupement arylène ou une chaîne alkylène linéaire ou ramifiée C₁-C₁₂ éventuellement substituée par un ou plusieurs groupements choisis parmi hydroxy, oxo, amino, SO₃H, CO₂H et dont un ou plusieurs des atomes de carbone est éventuellement remplacé par un atome d'oxygène, par un atome d'azote ou un groupement SO₂,
à l'exclusion du bis(aminométhanesulfonate) de strontium, du méthanedisulfonate de strontium, du 1,3-benzènedisulfonate de strontium, du 6-sulfonatohexanoate de strontium, du 1,5-naphtalènedisulfonate de strontium, du 1,6-naphtalènedisulfonate de strontium, du bis (6-hydroxy-2-naphtalènesulfonate) de strontium, du 2-oxo-1,3-propanedisulfonate de strontium, de l'hydroxyméthanesulfonate de strontium, du bis(4-aminobenzènesulfonate) de strontium, du bis (2-amino-benzènesulfonate) de strontium, du bis (3-aminobenzènesulfonate) de strontium, du 1,2-éthanedisulfonate de strontium et du 3-amino-3-carboxy-1-propanesulfonate chlorure de strontium,
étant entendu que par arylène, on entend phénylène, biphénylylène ou naphtylène, chacun de ces groupements étant éventuellement substitué par un ou plusieurs groupements, identiques ou différents, choisis parmi alkyle C₁-C₆ linéaire ou ramifié, OH, NH₂, SO₃H et CO₂H.

2. Sels de strontium selon la revendication 1, pour lesquels A représente SO₃H ou NH₂.

3. Sels de strontium selon la revendication 1 ou 2, pour lesquels B représente un groupement arylène ou une chaîne alkylène linéaire ou ramifiée C₁-C₁₂ éventuellement substituée par un ou plusieurs groupements choisis parmi hydroxy, oxo, amino, SO₃H, CO₂H et dont un ou plusieurs des atomes de carbone est éventuellement remplacé par un atome d'oxygène ou un groupement SO₂.

4. Sels de strontium selon la revendication 3, pour lesquels B représente une chaîne alkylène linéaire C₁-C₆ éventuellement substituée par un ou plusieurs groupements choisis parmi hydroxy, oxo, amino, SO₃H, CO₂H et dont un ou plusieurs des atomes de carbone est éventuellement remplacé par un atome d'oxygène ou un groupement SO₂.

5. Sels de strontium selon la revendication 4, pour lesquels B représente une chaîne alkylène linéaire C₃-C₆ éventuellement substituée par un ou plusieurs groupements choisis parmi hydroxy, oxo, amino, SO₃H, CO₂H et dont un ou plusieurs des atomes de carbone est éventuellement remplacé par un atome d'oxygène ou un groupement SO₂.

6. Sels de strontium selon la revendication 5, pour lesquels A représente un groupement choisi parmi NH₂ et SO₃H et B représente une chaîne alkylène linéaire ou ramifiée C₃-C₆ non substituée.

7. Sel de strontium selon la revendication 1, choisi parmi :
- le 1,3-propanedisulfonate de strontium;
- le bis(3-amino-1-propanesulfonate) de strontium;
- le sulfonatoacétate de strontium;
- le bis(2-amino-1-éthanesulfonate) de strontium;
- le 1,4-butanedisulfonate de strontium; et
- le 1,2-benzènedisulfonate de strontium.

8. Procédé de préparation d'un sel de strontium selon la revendication 1 par réaction d'un acide sulfonique de formule (I) :
A-B-SO₃H (I)
dans laquelle A et B sont tels que définis précédemment,
avec de l'hydroxyde de strontium, suivie de l'isolement du sel de strontium ainsi obtenu.

9. Procédé de préparation d'un sel de strontium selon la revendication 1 par réaction du sel de sodium ou de potassium d'un acide sulfonique de formule (I) :
A-B-SO₃H (I)
dans laquelle A et B sont tels que définis précédemment,
avec du chlorure de strontium, suivie de l'isolement du sel de strontium ainsi obtenu.

10. Composition pharmaceutique contenant comme principe actif le sel de strontium d'un acide sulfonique de formule (I) :
A-B-SO₃H (I)
dans laquelle :
- A représente un groupement choisi parmi OH, NH₂, SO₃H et CO₂H,
- B représente un groupement arylène ou une chaîne alkylène linéaire ou ramifiée C₁-C₁₂ éventuellement substituée par un ou plusieurs groupements choisis parmi hydroxy, oxo, amino, SO₃H, CO₂H et dont un ou plusieurs des atomes de carbone est éventuellement remplacé par un atome d'oxygène, d'azote ou un groupement SO₂,
en combinaison avec un ou plusieurs véhicules inertes, non toxiques et pharmaceutiquement acceptables,
étant entendu que par arylène, on entend phénylène, biphénylylène ou naphtylène, chacun de ces groupements étant éventuellement substitué par un ou plusieurs groupements, identiques ou différents, choisis parmi alkyle C₁-C₆ linéaire ou ramifié, OH, NH₂, SO₃H et CO₂H.

11. Utilisation du sel de strontium d'un acide sulfonique de formule (I) :
A-B-SO₃H (I)
dans laquelle :
- A représente un groupement choisi parmi OH, NH₂, SO₃H et CO₂H,
- B représente un groupement arylène ou une chaîne alkylène linéaire ou ramifiée C₁-C₁₂ éventuellement substituée par un ou plusieurs groupements choisis parmi hydroxy, oxo, amino, SO₃H, CO₂H et dont un ou plusieurs des atomes de carbone est éventuellement remplacé par un atome d'oxygène, d'azote ou un groupement SO₂,
pour la fabrication de médicaments utiles dans la prévention ou le traitement de l'arthrose,
étant entendu que par arylène, on entend phénylène, biphénylylène ou naphtylène, chacun de ces groupements étant éventuellement substitué par un ou plusieurs groupements, identiques ou différents, choisis parmi alkyle C₁-C₆ linéaire ou ramifié, OH, NH₂, SO₃H et CO₂H.

12. Utilisation du sel de strontium d'un acide sulfonique de formule (I) :
A-B-SO₃H (I)
dans laquelle :
- A représente un groupement choisi parmi OH, NH₂, SO₃H et CO₂H,
- B représente un groupement arylène ou une chaîne alkylène linéaire ou ramifiée C₁-C₁₂ éventuellement substituée par un ou plusieurs groupements choisis parmi hydroxy, oxo, amino, SO₃H, CO₂H et dont un ou plusieurs des atomes de carbone est éventuellement remplacé par un atome d'oxygène, d'azote ou un groupement SO₂,
pour la fabrication de médicaments utiles dans la prévention ou le traitement de l'ostéoporose,
étant entendu que par arylène, on entend phénylène, biphénylylène ou naphtylène, chacun de ces groupements étant éventuellement substitué par un ou plusieurs groupements, identiques ou différents, choisis parmi alkyle C₁-C₆ linéaire ou ramifié, OH, NH₂, SO₃H et CO₂H.

## Revendications modifiées

### Revendications modifiées conformément à la règle 86(2) CBE.

**1.** 1,3-Propanedisulfonate de strontium.

**2.** Procédé de préparation du 1,3-propanedisulfonate de strontium selon la revendication 1 par réaction d'acide 1,3-propanedisulfonique avec de l'hydroxyde de strontium, suivie de l'isolement du sel de strontium ainsi obtenu.

**3.** Procédé de préparation du 1,3-propanedisulfonate de strontium selon la revendication 1 par réaction du sel de sodium ou de potassium de l'acide 1,3-propanedisulfonique avec du chlorure de strontium, suivie de l'isolement du sel de strontium ainsi obtenu.

**4.** Composition pharmaceutique contenant comme principe actif le 1,3-propanedisulfonate de strontium selon la revendication 1, en combinaison avec un ou plusieurs véhicules inertes, non toxiques et pharmaceutiquement acceptables.

**5.** Utilisation du 1,3-propanedisulfonate de strontium selon la revendication 1 pour la fabrication de médicaments utiles dans la prévention ou le traitement de l'arthrose.
